# EUROPEAN PATENT APPLICATION

(11) **EP 1 514 582 A1**
(43) Date of publication of application: **16.03.2005**
(21) Application number: 04077676.7
(22) Date of filing: 14.09.2004
(51) Int. Cl.: A63B 21/06, A63B 21/068, A61H 33/06, A61N 5/06

(54) **Improved device for slimming down**

(30) Priority: 15.09.2003 BE 200300495
(71) Applicant: Jouck, Christa, 3550 Zolder (BE)
(72) Inventor: Jouck, Christa, 3550 Zolder (BE)
(74) Representative: Donné, Eddy

(57) **Abstract**

Improved device for slimming down, which mainly consists of a bed above which are provided infrared lamps and of means for training the muscles, characterised in that these means consist of a mass (24) provided in an upward guide and suspended onto a cable (23) which is guided over at least one cable pulley (20-21-22).

## Description

The present invention concerns an improved device for slimming down and improving the figure, as is known under the name body styling.

More particularly, the invention concerns a device for slimming down which mainly consists of a bed above which are provided infrared lamps and which is provided with means for training the muscles while using the device.

Such devices are known whereby the above-mentioned means consist of a spring which can be alternately tightened and released in order to train the muscles.

A disadvantage of such known devices is that the force required to tighten the spring is a function of the prolongation of the spring, whereby more particularly at the start of the movement, little force has to be exerted, whereas at the end of the movement this force becomes relatively large.

In practice it has been found that stressing the muscles in this way is not ideal to obtain a slimming effect.

Another disadvantage is that the spring may be overloaded, as a result of which it will lose its tensile force or it may even break.

Another disadvantage is that the use of such springs is relatively noisy.

The present invention aims to provide a solution to the above-mentioned and other disadvantages by providing a device which is equipped with simple means to train the muscles in an efficient manner, whereby the force to be exerted is always constant, which contributes to an efficient slimming process.

To this end, the invention concerns an improved device for slimming down which mainly consists of a bed above which are provided infrared lamps and of means for training the muscles, characterised in that these means consist of a mass provided in a vertical guide and which is suspended to a cable which is guided over at least one cable pulley.

An advantage is that the force which can be exerted to alternately move the force up and down is constant, which results in an efficient slimming process.

Another advantage is that the cable onto which a mass is suspended cannot be easily overloaded, as opposed to a spring.

The above-mentioned mass is preferably provided against a guide, such that it can be vertically moved, and the cable onto which the mass is suspended is guided over different cable pulleys, such that the cable onto which the mass is suspended also pushes said mass against the guide and simultaneously damps the movement of the mass.

This is advantageous in that the mass can be moved up and down noiselessly and without any oscillations.

More preferably, the improved device for slimming down is provided with four of the above-mentioned masses, which are each provided in a guide so that the arms and legs can be trained simultaneously. These guides are formed for example of struts over which is provided a hood onto which the above-mentioned infrared lamps are fixed.

This is advantageous in that the masses can be integrated in the existing struts in an aesthetical manner.

Also preferably, passages are provided in the bed at the height of the ankles and on either side at the height of the loins, through which the above-mentioned cables are guided between a few rollers which smoothly guide the cable in all directions.

In order to better explain the characteristics of the invention, the following preferred embodiment of an improved device according to the invention for slimming down is described as an example only without being limitative in any way, with reference to the accompanying drawings, in which:
figure 1 schematically represents a slimming device according to the invention in perspective;
figure 2 represents a section according to line II-II in figure 1;
figure 3 represents a section according to line III-III in figure 1;
figure 4 represents a section according to line IV-IV in figure 1;
figure 5 represents a detail of a part which is indicated by F5 in figure 4 in perspective.

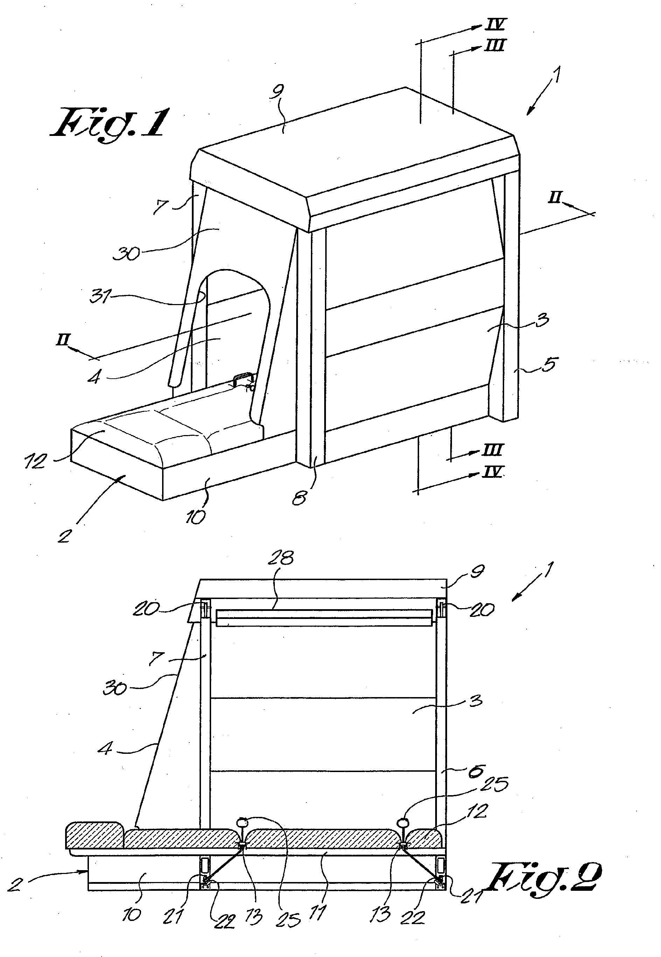

Figure 1 represents a device for slimming down according to the invention which is in this case made in the shape of a cabin 1 which mainly consists of a bed 2 which is partly surrounded by side walls 3-4 provided between struts 5-6-7-8, upon which rests a hood 9.

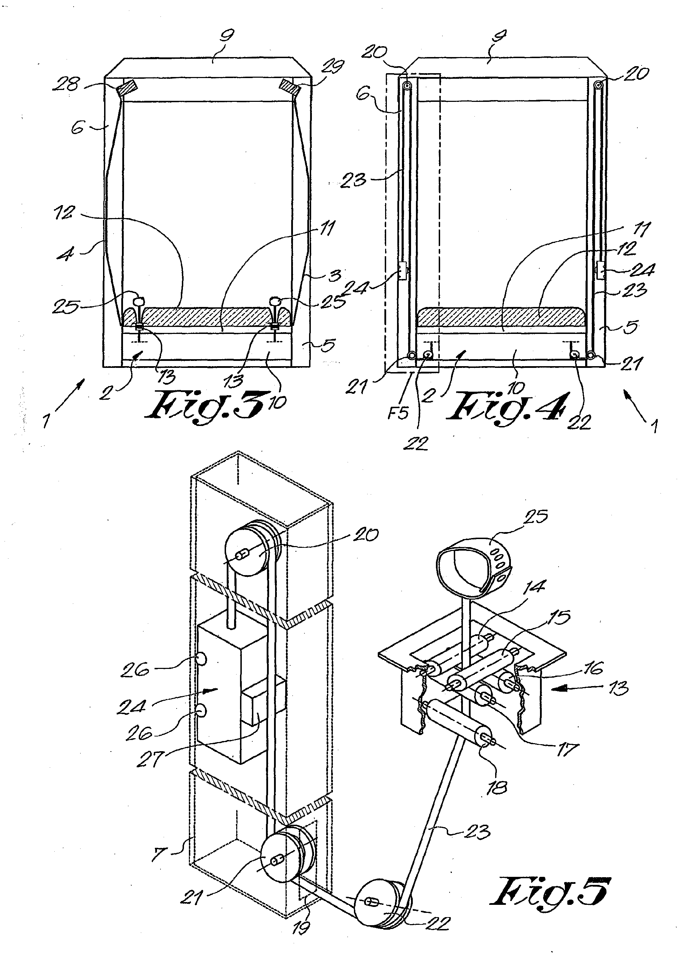

As represented in figures 1 to 4, the bed 2 is provided with a frame 10 in which has been provided a slatted base 11 or the like upon which rests a mattress 12.

Through the slatted base 11 and the mattress 12 are preferably provided four passages 13, whereby in each of the passages 13 are preferably provided five rollers 14-15-16-17-18, as represented in figure 5, and whereby the top four rollers 14-15-16-17 are diametrically opposed to each other in pairs. The fifth roller 18 is situated below the passage 13 concerned, more or less centrally under the above-mentioned four rollers 14-15-16-17.

Each of the rollers 14-15-16-17-18 is hereby provided such that they can freely rotate on shafts which are fixed, for example, in the slatted base 11.

The struts 5-6-7-8 are in this case made hollow and comprise each time one opening 19 in their side wall directed towards the inside of the cabin 1.

In struts 5-6-7-8 is provided at the top, above the level of the mattress 12, a first cable pulley 20, whereas at the bottom, under the level of the slatted base 11, in particular at the height of the opening 19, is provided a second cable pulley 21. Under the slatted base 11, next to each of the above-mentioned struts 5-6-7-8, is provided a third cable pulley 22.

Around the different cable pulleys 20-21-22, in and next to each strut 5-6-7-8, is each time guided a cable 23, onto which is suspended a mass 24 at one far end which can move freely up and down in a strut 5-6-7 or 8 concerned, and which preferably has a width which is almost equal to the width of the strut 5-6-7-8 concerned.

The free end of the above-mentioned cables 23 is led between the rollers 14-15-16-17 in the passages 13 in the bed 2 and is provided with a bangle 25 or the like which makes it possible to tie the cable 23 to the arms or legs of a user.

The mass 24 is provided with journals 26 or the like on one side, and it is provided with a plate 27 on its other side, which journals 26 and plate 17 are made of a material having a low frictional coefficient.

Finally, to the hood 9 are fixed infrared lamps 28-29 which are directed towards the bed 2.

The use of the device according to the invention is simple and as follows.

A user takes place on the bed 2 in the cabin 1 and under the lamps 28-29. Next, he fixes the bangles 25 on the free ends of the different cables 23 to his wrists and ankles.

As soon as the user is lying down, the infrared lamps 28-29 are switched on, and the user can alternately move his arms and legs to and fro, whereby the user has to make an effort to move the masses up and down.

The effect of such a use of the device is an efficient use of energy by the user, who will consequently slim down fast, whereby the lamps produce the required warmth to promote sweating, and the regularly exerted force burns the necessary calories, such as fats and the like.

The positioning of the cable pulleys 20-21 is preferably such that the part of the cable which spans the cable pulleys 20-21, pushes the mass 24 concerned against the inner side of the struts 5-6-7-8, so that an oscillation of the mass 24 is prevented and the mass moves almost noiselessly in the strut 5-6-7 or 8.

Also preferably, the cabin 1 is sealed by a front wall 30 having a passage 31 for the body and a back wall which is not represented in the figures.

Although, according to the most preferred embodiment, the free ends of the cables 23 are guided through the passages 13 in the bed 2, it is also possible, of course, to move the cables 23 via another way into cabin 1, for example via one of the side walls, through the hood 9 or the like.

The present invention is by no means limited to the embodiment given as an example and represented in the accompanying drawings; on the contrary, such a device for slimming down according to the invention can be made in all sorts of shapes and dimensions while still remaining within the scope of the invention.

## Claims

1. Improved device for slimming down, which mainly consists of a bed above which are provided infrared lamps and of means for training the muscles, **characterised in that** these means consist of a mass (24) provided in an upward guide and suspended onto a cable (23) which is guided over at least one cable pulley (20-21-22).

2. Improved device according to claim 1, **characterised in that** the above-mentioned upward guide consist of a hollow strut (5-6-7-8), in which the above-mentioned mass (24) has been provided, whereby the width of the mass (24) is almost equal to the width of the strut (5-6-7-8) concerned.

3. Improved device according to claim 2, **characterised in that** in the above-mentioned struts (5-6-7-8) are provided at least two cable pulleys (20-21) over which the above-mentioned cable (23) is guided.

4. Improved device according to claim 3, **characterised in that** the mass (24) which is suspended onto the cable (23) is situated at a level between the above-mentioned cable pulleys (20-21), whereby the part of the cable (23) spanning the two cable pulleys (20-21) pushes against the mass (24).

5. Improved device according to claim 4, **characterised in that** the part of the cable (23) spanning the cable pulleys (20-21) pushes the mass (24) against the strut (5-6-7-8) concerned.

6. Improved device according to any of claims 4 and 5, **characterised in that** on the spot where the cable (23) pushes against the mass (24), a plate (27) is fixed to the mass (24), which plate (27) is made of a material having a low frictional coefficient.

7. Improved device according to claim 6, **characterised in that** the mass (24), on the sides making contact with the strut (5-6-7-8), is provided with at least one journal (26) .

8. Improved device according to any one of the preceding claims, **characterised in that** under the bed (2), next to the above-mentioned struts (5-6-7-8), is provided a third cable pulley (22).

9. Improved device according to any one of the preceding claims, **characterised in that** in the bed (2) are provided passages (13) through which the free end of the cable (23) protrudes.

10. Improved device according to claim 9, **characterised in that** in the above-mentioned passages (13) are provided freely rotating rollers (14-15-16-17-18) in between which the cable (23) is guided.

11. Improved device according to claim 10, **characterised in that** in the above-mentioned passages (13) are each time provided four rotating rollers (14-15-16-17) which are diametrically opposed in pairs.

12. Improved device according to claim 11, **characterised in that** below the passage (13) is provided a fifth roller (18) which is situated more or less centrally under the above-mentioned four rollers (14-15-16-17) .

13. Improved device according to any one of the preceding claims, **characterised in that** it is made in the shape of a cabin (1), with a bed (2) and a hood (9) with infrared lamps (28-29), whereby the hood (9) is provided on four struts (5-6-7-8), and whereby a mass (24) is provided in each strut (5-6-7-8) which is suspended to a cable (23) which opens into the cabin (1) with its free end.
